# EUROPÄISCHE PATENTSCHRIFT

(11) **EP 1 014 902 B1**
(45) Veröffentlichungstag und Bekanntmachung des Hinweises auf die Patenterteilung: **25.11.2009**
(21) Anmeldenummer: 98950052.5
(22) Anmeldetag: 18.09.1998
(51) Int. Cl.: A61F 9/007, A61F 9/00

(54) **VORRICHTUNG FÜR EINEN ZUGANG IN DEN SUBRETINALRAUM EINES AUGES**
DEVICE FOR ACCESSING THE SUB-RETINAL SPACE OF AN EYE
DISPOSITIF PERMETTANT UN ACCES A L'ESPACE SOUS-RETINIEN D'UN OEIL

(30) Priorität: 19.09.1997 DE 19741487
(43) Veröffentlichungstag der Anmeldung: 05.07.2000
(73) Patentinhaber: Retina Implant AG, 72770 Reutlingen (DE)
(72) Erfinder: GABEL, Veit-Peter, D-93049 Regensburg (DE); KOBUCH, Karin, D-93080 Pentling (DE); ZRENNER, Eberhart, D-72076 Tübingen (DE)
(74) Vertreter: Gahlert, Stefan
(86) Internationale Anmeldenummer: PCT/EP1998/005953
(87) Internationale Veröffentlichungsnummer: WO 1999/015119

(56) Entgegenhaltungen:
- EP-A- 0 460 320
- US-A- 4 641 648
- US-A- 4 722 724
- US-A- 4 747 393
- US-A- 5 370 652
- US-A- 5 507 807
- US-A- 5 578 040

## Beschreibung

Die Erfindung betrifft eine Vorrichtung für einen Zugang in den Subretinalraum eines Auges, mit einem langgestreckten flachen Körper aus weichem Material, der seitlich durch einen Einschnitt in der Sclera des Auges in den Subretinalraum einschiebbar ist.

Eine Vorrichtung der vorstehend genannten Art ist aus der EP 0 460 320 A2 bekannt.

Die bekannte Vorrichtung wird im Zusammenhang mit dem Einbringen eines Retina-Implantats verwendet. Sie besteht aus einer Baugruppe, die einen flachgedrückten Kunststoffschlauch sowie einen in dem Kunststoffschlauch befindlichen Schieber umfaßt. Zum Einbringen des Implantats wird dieses in Gestalt eines kreisförmigen, flachen Mikrochips in die Nähe der vorderen Öffnung des Schlauches eingebracht. Hinter das Implantat wird der Schieber in den Schlauch eingeführt. Diese Baugruppe wird nun durch einen Einschnitt im Auge eingeführt, bis sich das freie Ende des Schlauches an der gewünschten Implantatposition befindet. Der Schlauch wird dann zurückgezogen, während der Schieber raumfest gehalten wird, so daß auf diese Weise das Implantat an seine vorgesehene Stelle gelangt.

Zum Einbringen des Implantats werden zwei unterschiedliche Operationsmethoden vorgeschlagen. Bei der einen Operationsmethode wird ein Schnitt im Bereich der Pars plana eingebracht und das Implantat durch den Glaskörper des Auges hindurchgeführt und dann durch einen Schnitt, der auf der Glaskörperseite der Netzhaut angebracht wird, in den Subretinalraum geschoben.

Bei einer anderen Operationsmethode wird ein Schnitt durch die Sclera unmittelbar hinter der Ora serrata angebracht. Der Schnitt wird dann durch die Chorioidea, die Choriocapillaris, die Bruch'sche Membran sowie das Pigmentepithel der Netzhaut geführt, so daß das Implantat zwischen den inneren und den äußeren Schichten der Netzhaut eingebracht werden kann.

Die bekannte Vorrichtung hat jedoch den Nachteil, daß sie verhältnismäßig dick ausgebildet ist und eine Kontrolle der Positionierung kaum möglich ist. Die erhebliche Dicke der Anordnung bestehend aus dem flachen Schlauch und dem Schieber ist bei dem genannten Einsatzfall im Subretinalraum besonders störend, weil sowohl die Netzhaut (Retina) wie auch die Aderhaut (Chorioidea) äußerst empfindlich sind und die Aderhaut darüber hinaus zu starken Blutungen neigt. Die mangelnde Möglichkeit, eine genaue Positionskontrolle vorzunehmen, ist ebenfalls für diesen Einsatzfall sehr störend, weil Retina-Implantate genau positioniert werden müssen und weil es auch bei anderen Anwendungsfällen, die einen Zugang zum Subretinalraum erfordern, wichtig ist, die genaue Position zu kennen.

Aus der US 5,578,040 ist eine Vorrichtung von weitgehend röhrenförmiger Gestalt bekannt, in der eine medizinische Vorrichtung an eine gewünschte Position gebracht werden kann.

Der Erfindung liegt daher die Aufgabe zugrunde, eine Vorrichtung der eingangs erwähnten Art dahingehend weiterzubilden, daß die geschilderten Nachteile vermieden werden. Insbesondere soll eine besonders schonende Behandlung im Bereich des Zugangsweges erreicht werden, und es soll ferner ermöglicht werden, die Positionierung des Zuganges genau kontrollieren zu können.

Diese Aufgabe wird dadurch gelöst, daß ein Operationskit bereitgestellt wird, das einen langgestreckten flachen Streifen aus weichem Material und einen Schieber zum Verschieben des medizinischen Gerätes unter Führung des langgestreckten flachen Streifens, wobei der Streifen als Folie ausgebildet ist, die eine äußere Oberfläche aufweist, die als Führungsfläche für das medizinische Gerät ausgebildet ist, wobei sowohl die Folie als auch der Schieber in ihren Abmessungen derart ausgebildet sind, daß ein Einführen des medizinischen Gerätes durch einen seitlichen Einschnitt in der Sclera in den Subretinalraum des Auges ermöglicht ist.

Die der Erfindung zugrunde liegende Aufgabe wird auf diese Weise vollkommen gelöst. Die Verwendung eines Streifens hat nämlich den Vorteil, daß eine besonders flache Bauweise möglich ist, während andererseits die Führungsfunktion in vollem Umfang erhalten bleibt. Es hat sich nämlich gezeigt, daß die im Stand der Technik bekannte Führung an mehreren Führungsoberflächen nicht nur bei vielen Anwendungsfällen unnötig ist, sie birgt darüber hinaus auch die Gefahr eines Verkantens oder Verklemmens, abgesehen von den bereits erwähnten Nachteilen, die die große Baugröße, insbesondere die Dicke der Vorrichtung, mit sich bringt.

Bei einer bevorzugten Ausgestaltung der Erfindung ist die Folie mit einer Dicke von etwa 30 bis 70 µm, vorzugsweise etwa 50 µm, ausgebildet.

Diese Maßnahme hat den Vorteil, daß eine extrem dünne Bauweise möglich ist, gleichzeitig aber die gewünschte Führungsfunktion in vollem Umfange gewährleistet werden kann.

Weiterhin ist erfindungsgemäß bevorzugt, die Folie mit einer Breite von etwa 1 bis 5 mm, vorzugsweise etwa 2 mm, sowie einer Länge von etwa 15 bis 40 mm, vorzugsweise etwa 25 mm, zu versehen.

Bei weiteren bevorzugten Ausgestaltungen der Erfindung besteht die Folie aus Kunststoff, vorzugsweise Polyethylen oder Polypropylen.

Bei einer weiteren besonders bevorzugten Ausführungsform der Erfindung ist mindestens die Folie mit einer Skalierung versehen.

Diese Maßnahme hat den Vorteil, daß eine genaue Positionskontrolle möglich ist. Diese Positionskontrolle kann dadurch geschehen, daß die Skalierung relativ zum Einschnitt in der Sclera abgelesen wird, man kann aber auch die Skalierung relativ zur Position des geführten medizinischen Gerätes ablesen, insbesondere dann, wenn auch dieses mit einer Skala oder einer entsprechenden Markierung versehen ist.

Obwohl die Erfindung nachstehend anhand eines Ausführungsbeispiels beschrieben werden wird, das das Einbringen von Implantaten in den subretinalen Raum zum Gegenstand hat, versteht sich, daß die Erfindung auch auf vielen anderen Gebieten einsetzbar ist.

So kann die Erfindung vorzugsweise bei einem Operationsgerät für subretinale Eingriffe im Auge eingesetzt werden, insbesondere dadurch, daß auf der Führungsfläche der Folie ein Retina-Implantat mittels eines Schiebers in den Subretinalraum einführbar ist.

Bei diesem Operationsgerät kann auf der Führungsfläche die Folie aber auch ein mikrochirurgisches Instrument in den Subretinalraum einführbar sein, wobei das mikrochirurgische Gerät vorzugsweise ein Laser ist.

Bei einem anderen Einsatzfall der Erfindung handelt es sich um ein Mikroendoskop für den Subretinalraum eines Auges, bei dem eine erfindungsgemäße Vorrichtung verwendet wird. Entsprechendes gilt für ein Gerät zum Einbringen eines mit einem Wirkstoff versehenen Trägers in den Subretinalraum eines Auges.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegeben Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: in perspektivischer Seitenansicht ein herauspräpariertes Auge;

- Fig. 2 und 3: vergrößerte Detaildarstellungen eines Bereiches des Auges aus Fig. 1 zur Veranschaulichung von zwei Schritten eines Verfahrens, bei dem die Erfindung vorzugsweise einsetzbar ist;
- Fig. 4: einen schematisierten Querschnitt durch ein Auge.

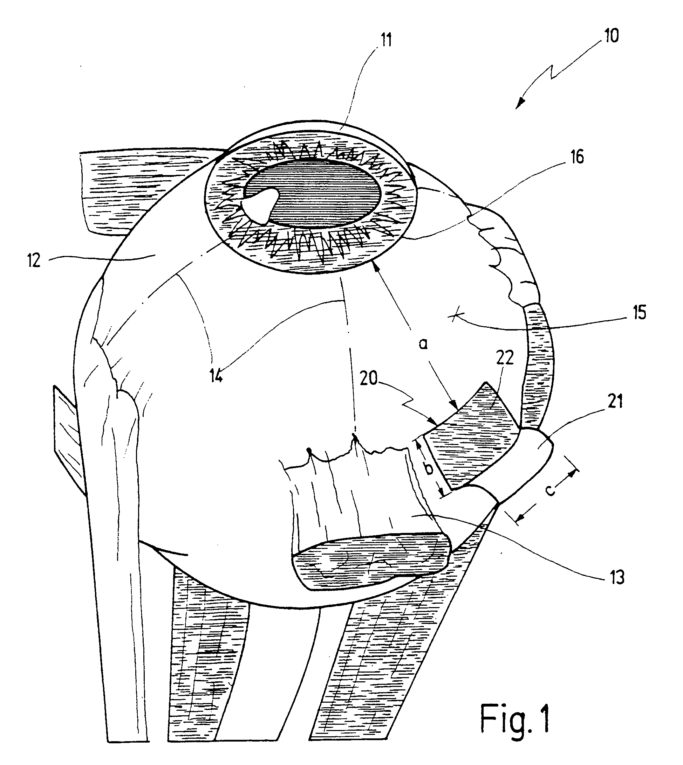

In den Figuren bezeichnet 10 einen Augapfel. Der Augapfel 10 weist an seiner Vorderseite eine Hornhaut (Cornea) 11 und im übrigen eine Lederhaut (Sclera) 12 auf. In Fig. 1 ist vorne am Augapfel 10 der Musculus rectus superior 13 zu erkennen. Mit strichpunktierten Linien 14 sind auf der Oberfläche des Augapfels 10 vier Quadranten eingezeichnet. Der hier interessierende äußere, obere Quadrant ist mit 15 bezeichnet. Der Limbus corneae, d.h. der Rand der Hornhaut 11, ist in Fig. 1 bei 16 eingezeichnet.

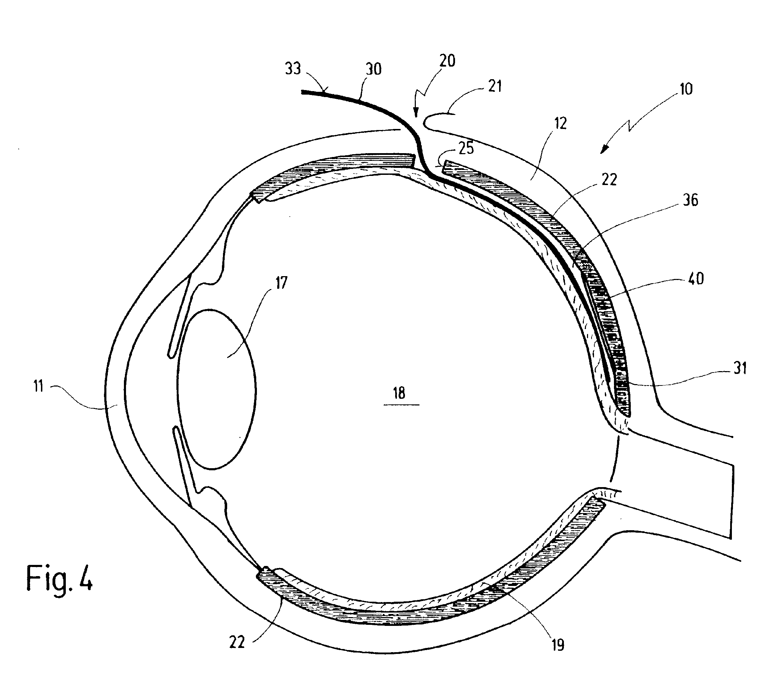

Fig. 4 zeigt noch die Linse 17 sowie den Glaskörper 18 des Auges, hinter dem sich die Netzhaut (Retina) 19 befindet.

Während man bei herkömmlichen Operationen im Subretinalraum einen Zugang durch den Glaskörper 18 und durch die Netzhaut 19 hindurch wählt, ist im Rahmen der vorliegenden Erfindung vorgesehen, eine Sclerainzision 20 im Bereich des äußeren, oberen Quadranten 15 vorzunehmen, wie deutlich aus Fig. 1 erkennbar. Die Sclerainzision 20 wird rechteckförmig ausgeführt, und zwar in einem Abstand a von vorzugsweise 7 bis 8 mm vom Limbus corneae. Durch die Sclerainzision 20 wird ein Scleralappen 21 einer Breite b und einer Länge c ausgeschnitten, der vorzugsweise 4 x 4 mm groß ist. Unterhalb des Scleralappens 21 wird die Aderhaut (Chorioidea) 22 sichtbar.

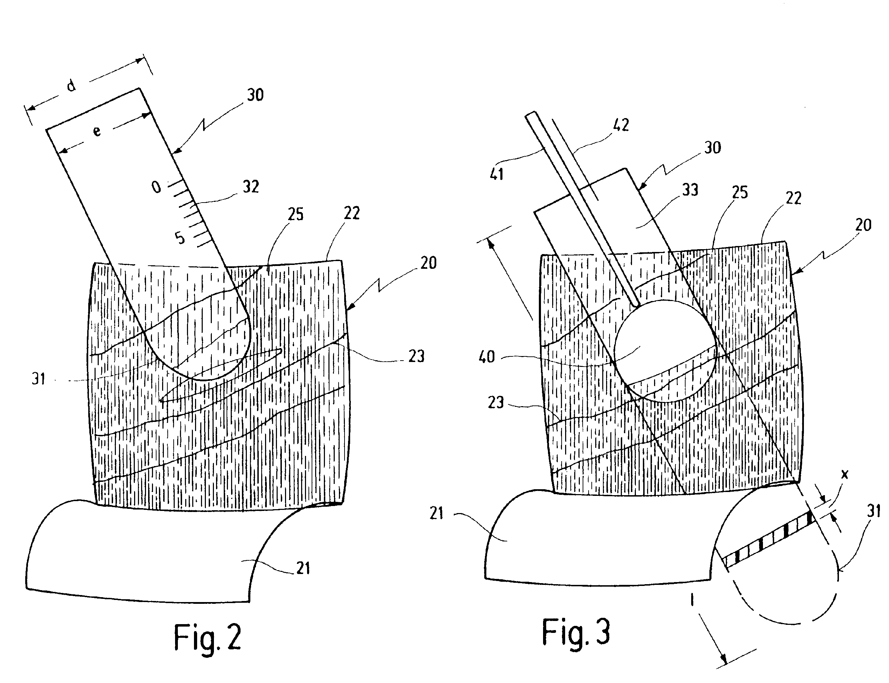

Wie aus den vergrößerten Darstellungen gemäß Fig. 2 und 3 erkennbar ist, wird nun in die Aderhaut 22, und zwar vorzugsweise parallel zu den chorioidealen Gefäßen 23, ein Schnitt 25 durch die Aderhaut 22 hindurch angebracht. Der Schnitt 25 hat eine Breite d von vorzugsweise 2,5 bis 3,5 mm.

Durch den Schnitt 25 kann nun ein Folienstreifen 30 geschoben werden, der eine etwas geringfügigere Breite e von z.B. 2 mm hat. Der Folienstreifen 30 hat darüber hinaus eine Länge l von vorzugsweise 25 mm und eine Dicke x von nur 50 µm. Er ist an seinem vorderen Ende 31 abgerundet.

Der Folienstreifen 30 kann mit einer Skalierung 32 versehen sein, um die Eintauchtiefe des Folienstreifens 30 im Schnitt 25 unmittelbar ablesen zu können.

Eine Oberfläche 33 des Folienstreifens 30 dient als Führungsfläche, wie noch erläutert werden wird.

Aus der Querschnittsdarstellung gemäß Fig. 4 kann man erkennen, daß der Folienstreifen 30 durch die Sclerainzision 20 und den Schnitt 25 in der Aderhaut 22 hindurch in den Subretinalraum 36 hineingeschoben wurde. Mittels eines Schiebers 41 (Fig. 3) kann nun ein Implantat, beispielsweise ein Multiphotodiodenarray 40, in den Subretinalraum 36 eingeschoben werden. Als Schieber 41 kann hierzu vorzugsweise ein Kunststoffschlauch verwendet werden. Der Schieber 41 kann ebenfalls mit einer Skala versehen sein. Die Schieberichtung ist in Fig. 3 mit 42 bezeichnet.

Mit der beschriebenen Vorrichtung kann eine sogenannte "ab externo" Implantation eines Implantats wie folgt ausgeführt werden:

Nach Bindehautschnitt und Präparation der Bulbuswand im äußeren oberen Quadranten 15 werden der Musculus rectus superior 13 und lateralis als Haltezügel angeschlungen. Im Abstand a von 8 mm vom Limbus corneae 16 wird temporal oben ein Scleralappen 21 von 4 mm Kantenlänge präpariert. Die Sclerainzision 20 kann nach Stand der Erfahrung des Operateurs und Übersichtlichkeit des Operationsgebietes auf einen Schnitt von 6 mm Länge in Richtung des mutmaßlichen Verlaufs der chorioidealen Gefäße 21 reduziert werden.

Durch eine Parazentese wird nun der intraokuläre Druck so weit abgesenkt, daß sich die Chorioidea 22 im Bereich des Scleralappens 21 nicht mehr vorwölbt.

Um eine lokale Vasokonstriktion zu erreichen, wird ein Tropfen Ornipressin (Verdünnung 0,5 I.E./ml) auf die Chorioidea 22 aufgebracht. Die Chorioidea 22 wird anschließend auf eine Länge d von 2 mm entlang dem Verlauf der großen Gefäße 23 inzidiert. Der vorzugsweise 2 mm breite und vorne abgerundete Folienstreifen 30 wird auf die sich vorwölbende neurosensorische Netzhaut 19 aufgelegt, bis zum Rand der Chorioidektomie zurückgezogen und dann in den Subretinalraum 36 vorgeschoben.

Das Implantat, nämlich der Mikrophotodiodenchip 40, wird auf den Folienstreifen 30 aufgelegt und entlang des Folienstreifens 30 in den Subretinalraum 36 eingeführt. Die subretinale Lage des Chips 40 ist aus der Implantationsrichtung entlang dem Folienstreifen 30 und der Länge der implantierten Folie ableitbar, ohne direkten intraokulären Einblick.

Die Folie wird anschließend aus dem Subretinalraum 36 zurückgezogen, zum Schutz von Netzhaut 19 und Chorioidea 22 und zur Vermeidung von Netzhautinkarzeration im Inzisionsbereich aber weiterhin auf Netzhaut 19 und Chorioidea 22 belassen, während die vorgelegten Scleranähte verschlossen werden.

Durch die beschriebene Operation ist ein direkter, transscleraler, transchorioidaler Zugang zum Subretinalraum ohne Eröffnung des Intraokularraumes möglich. Auf diese Weise können Operationsrisiken von Vitrektomie und Retinotomie weitgehend vermieden werden, insbesondere ein Katarakt, eine Ablatio und PVR.

Der erfindungsgemäß mögliche Zugang zum Subretinalraum kann auch bei anderen klinischen Fragestellungen verwendet werden, z.B. bei subretinalen Neovaskularisationen, Blutungen und Membranen, Implantationen und Explantationen von Mikrophotodiodenchips, Transplantationen von Pigmentepithel und Netzhautgewebe sowie bei der subretinalen Applikation von Medikamenten. Eine gezielte Plazierung des Chips bzw. von Medikamenten oder Mikroinstrumenten ab externo an eine definierte Stelle im Subretinalraum ist auf diese Weise auch ohne direkten intraokulären Einblick möglich.

Bei einer anderen klinischen Anwendung kann über den externen, oben beschriebenen Zugang auf der Folie ein Mikroendoskop von beispielsweise 0,9 mm Durchmesser, einschließlich Spülkanal und Option für die Integration von Laser oder Mikrofaßzange, in den Subretinalraum eingeführt werden. Bei geringer Spülung mit Kochsalzlösung ist mit einer solchen Lichtfaseroptik (6.000 Pixel) der Chip im Subretinalraum darstellbar. Man kann auf diese Weise z.B. die genaue Lage des Chips feststellen, diesen gegebenenfalls austauschen oder andere mikrochirurgische Eingriffe unter subretinaler endoskopischer Kontrolle durchführen, wenn Mikroinstrumente in das Endoskop integriert sind, beispielsweise Scherchen, Faßzangen oder Laser.

## Patentansprüche

1. Operationskit zum Einführen eines medizinischen Gerätes in ein Auge (10), umfassend einen langgestreckten flachen Streifen aus weichem Material und einen Schieber zum Verschieben des medizinischen Gerätes unter Führung des langgestreckten flachen Streifens (30), **dadurch gekennzeichnet, daß** der Streifen als Folie ausgebildet ist, die eine äußere Oberfläche aufweist, die als Führungsfläche für das medizinische Gerät ausgebildet ist, und daß sowohl die Folie als auch der Schieber in ihren Abmessungen derart ausgebildet sind, daß ein Einführen des medizinischen Gerätes durch einen seitlichen Einschnitt (20) in der Sclera (12) in den Subretinalraum (36) des Auges ermöglicht ist.

2. Operationskit nach Anspruch 1, **dadurch gekennzeichnet, daß** die Folie (30) mit einer Dicke (x) von etwa 30 bis 70 µm, vorzugsweise 50 µm, ausgebildet ist.

3. Operationskit nach Anspruch 1 oder 2, **dadurch gekennzeichnet, daß** die Folie (30) eine Breite (e) von etwa 1 bis 5 mm, vorzugsweise 2 mm, aufweist.

4. Operationskit nach einem oder mehreren der Ansprüche 1 bis 3, **dadurch gekennzeichnet, daß** die Folie (30) eine Länge (1) von etwa 15 bis 40 mm, vorzugsweise etwa 25 mm, aufweist.

5. Operationskit nach einem oder mehreren der Ansprüche 1 bis 4, **dadurch gekennzeichnet, daß** die Folie (30) aus Kunststoff, vorzugsweise Polyethylen oder Polypropylen besteht.

6. Operationskit nach einem oder mehreren der Ansprüche 1 bis 5, **dadurch gekennzeichnet, daß** mindestens die Folie (30) mit einer Skalierung (32) versehen ist.

## Claims

1. An operation kit for introducing a medical device into an eye (10), comprising an elongated flat strip of soft material and a slider for sliding the medical device while being guided by the elongated flat strip (30), **characterized in that** the strip is formed as a foil, the foil comprising an outer surface formed as a guiding surface for the medical device, and **in that** both the foil and the slider are formed with dimensions, such, that introduction of the medical device into the subretinal region (36) of the eye is enabled through a side incision (20) within the sclera (12).

2. The operation kit according to claim 1, **characterized in that** the foil (30) is formed with a thickness (x) of about 30 to 70 µm, preferably 50 µm.

3. The operation kit according to claim 1 or 2, **characterized in that** the foil (30) has a width (e) of about 1 to 5 mm, preferably 2 mm.

4. The operation kit according to anyone or several of claims 1 to 3, **characterized in that** the foil (30) has a length (1) of about 15 to 40 mm, preferably about 25 mm.

5. The operation kit according to anyone or several of claims 1 to 4, **characterized in that** the foil (30) is made of plastic, preferably polyethylene or polypropylene.

6. The operation kit according to anyone of several of claims 1 to 5, **characterized in that** at least the foil (30) is provided with a scale (32).

## Revendications

1. Kit d'opération permettant d'introduire un dispositif médical à l'intérieur de l'oeil (10), comportant une rayure plate allongée en un matériau souple et un élément coulissant permettant de déplacer le dispositif médical en guidant la rayure plate allongée (30), **caractérisé en ce que** la rayure est configurée comme une feuille, qui présente une surface extérieure configurée comme une surface de guidage pour le dispositif médical, et **en ce que** la feuille tout comme l'élément coulissant ont des dimensions telles que l'introduction du dispositif médical à travers une incision latérale (20) dans la sclère (12) est rendue possible dans l'espace sous-rétinien (36) de l'oeil.

2. Kit d'opération selon la revendication 1, **caractérisé en ce que** la feuille (30) est configurée avec une épaisseur (x) allant d'environ 30 à 70 µm, de préférence une épaisseur de 50 µm.

3. Kit d'opération selon la revendication 1 ou 2, **caractérisé en ce que** la feuille (30) présente une largeur (e) allant d'environ 1 à 5 mm, de préférence une largeur de 2 mm.

4. Kit d'opération selon l'une quelconque ou plusieurs des revendications 1 à 3, **caractérisé en ce que** la feuille (30) présente une longueur (1) allant d'environ 15 à 40 mm, de préférence une longueur d'environ 25 mm.

5. Kit d'opération selon l'une quelconque ou plusieurs des revendications 1 à 4, **caractérisé en ce que** la feuille (30) se compose de matière plastique, de préférence de polyéthylène ou de polypropylène.

6. Kit d'opération selon l'une quelconque ou plusieurs des revendications 1 à 5, **caractérisé en ce qu'**au moins la feuille (30) est munie d'une graduation (32).
